# EUROPEAN PATENT APPLICATION

(11) **EP 0 645 152 A2**
(43) Date of publication of application: **29.03.1995**
(21) Application number: 94202780.6
(22) Date of filing: 27.09.1994
(51) Int. Cl.: A61M 5/24, A61M 5/32, A61M 5/50, A61M 5/00, A61M 5/315

(54) **Disposable holder for pre-filled cartridge-needle unit**

(30) Priority: 29.09.1993 US 129878
(71) Applicant: STERLING WINTHROP INC., New York, NY 10016 (US)
(72) Inventor: Stiehl, Mark A., Rochester, New York 14650 (US); Bergstresser, William A., Rochester, New York 14650 (US); Chapman, Raymond P., Rochester, New York 14650 (US); Niedospial, John J., Sterling Winthrop Inc., New York 10016 (US)
(74) Representative: Le Guen, Gérard

(57) **Abstract**

This invention relates to a holder for use in combination with pre-filled cartridge-needle units having a proximal end and a needle (22) at the distal end thereof, the holder comprising a hollow body (36) sized for housing the cartridge-needle unit therein, means for permitting the body (36) to move axially relative to the cartridge-needle unit and for retaining the cartridge-needle unit within the holder; and a cap (40) for the proximal end of the cartridge-needle unit, wherein the cartridge-needle unit can be activated by application of force or torque to the cap (40). In a preferred embodiment, the cap (40) is tamper-evident and attached to the proximal end of the body (36). The holder reduces the susceptibility of health care workers to accidental needle strikes and enables the cartridge-needle unit to be activated safely and easily.

## Description

This invention relates to a disposable holder for pre-filled cartridge-needle units containing means for activating the cartridge-needle unit.

Disposable medicament-containing cartridge needle units for use in conjunction with reusable hypodermic syringe holders are well known in the art and in widespread commercial use. Such cartridges conventionally feature a cylindrical body closed at the proximal end with a flexible plunger slidable within the bore of the cartridge and closed at the distal necked-down end with a septum secured to the cartridge by a crimped-on aluminum collar. The necked-down distal end conventionally is fitted with a needle hub/needle/needle guard assembly. Such cartridge-needle units are available from Sanofi Winthrop Pharmaceuticals under the Carpuject® trademark.

These units have satisfied widespread demand. However, on occasion, and for reasons which are not completely understood, a very small percentage of these units have experienced so-called 'plunger backout', i.e. the plunger slides towards the proximal end of the cartridge, sometimes exposing the sterile solution and thus rendering it unsuitable for use. Plunger backout tends to be exacerbated when the units are transported via air or used and/or stored in high altitude environments. Additionally, these units can be subject to unauthorized diversion of the medicaments contained therein.

In use, the cartridge-needle unit must be activated, i.e. the proximal end of the needle cannula must penetrate the sealed septum such that communication is achieved between the fluid and the proximal end of the needle. When used in conjunction with conventional reusable syringe holders of the type described, for example, in Hadtke, U.S. Patent 4,585,445 and in EP-A-0 485,028, this is accomplished when the health care worker advances the cartridge through the holder by rotating a clamping element.

Such reusable holders enable the user to avoid holding the cartridge-needle unit when the needle unit is exposed. Nevertheless, health care workers are especially susceptible to accidental and potentially infectious, and indeed, on occasion, possibly fatal, needle strikes due to the careless handling and/or disposing of the cartridge-needle unit after use. The consequences to health care workers of strikes from needles contaminated with various infectious diseases such as hepatitis or AIDS can be particularly severe. The frequency of such accidental needle strikes in the United States is surprisingly great, and has been estimated to be approximately one million strikes per year. Moreover, the cost to health care organizations for the testing of health care workers accidentally stricken by used needles is a significant burden on health care costs. Therefore, it would be desirable to further protect health care workers by providing systems which reduce the possibility of accidental needle strikes.

To this end, it has been suggested to provide a hollow body to house the cartridge-needle unit, such that the needle can be exposed for use and then withdrawn into the body for safety. For example, PCT/US91/05613 describes disposable, i.e. single use, holders which can be used in conjunction with cartridge-needle units. PCT/US91/06878 describes another such system featuring a holder containing a radially deformable body. However, both of these systems require that the user activate the cartridge by pressing the proximal end of the exposed glass cartridge-needle unit. This leads to the possibility that sharp or broken glass can pose a safety risk to the user. Moreover, the activation process can require the user to generate up to as much as about 4000 kPa (600 PSI [22.7 lb force over an area of 0.03677 square inches]). Consequently, these systems are less than fully satisfactory from a commercial standpoint.

In addition, existing safety syringe systems often are not economical to produce. This is especially true when the syringe system is intended to be disposable.

It would be desirable to provide a disposable holder for use with a cartridge-needle unit which enables the unit to be activated safely and easily and which provides the safety advantage of enabling the exposed needle to be withdrawn into the body of the holder after use.

According to the present invention there is provided a disposable holder having all the above features for use in combination with pre-filled cartridge-needle units having a proximal end and a needle at the distal end thereof, the holder comprising
a hollow body sized for housing the cartridge-needle unit therein;
means for permitting the body to move axially relative to the cartridge-needle unit and for retaining the cartridge-needle unit within the holder; and
a cap for the proximal end of said cartridge-needle unit wherein the cartridge-needle unit can be activated by application of force to the cap.

In another embodiment of the invention, the disposable holder comprises
a hollow body sized for housing the cartridge-needle unit therein; and
a tamper-evident cap for the proximal end of such cartridge-needle unit attached to the proximal end of the body, wherein the cartridge-needle unit can be activated by application of force to the cap.

It is an advantageous feature of this invention that a holder is provided for cartridge-needle units which enables the unit to be activated by health care workers easily and safely.

It is another advantageous feature of this invention that a holder is provided for cartridge-needle units which can be used safely and effectively with reduced exposure of the user to the needle cannula. This reduces the susceptibility of health care workers to accidental needle strikes.

Still another advantage of this invention is that a disposable, i.e. single use, holder is provided for commercially available cartridge-needle units.

Yet another advantageous feature of this invention is that a holder is provided which can be easily and economically manufactured and which evidences any unauthorized diversion of the medication in the associated cartridge-needle unit.

A further advantageous feature of this invention is that a holder-cartridge needle unit system is provided which entirely eliminates the 'plunger backout' problem described above.

The invention will now be described with reference to the following Figures but is in no way to be construed as limited thereby.

Figures 1 and 2 are front and side views of a preferred embodiment of the holder of this invention, an associated plunger rod and a cap.

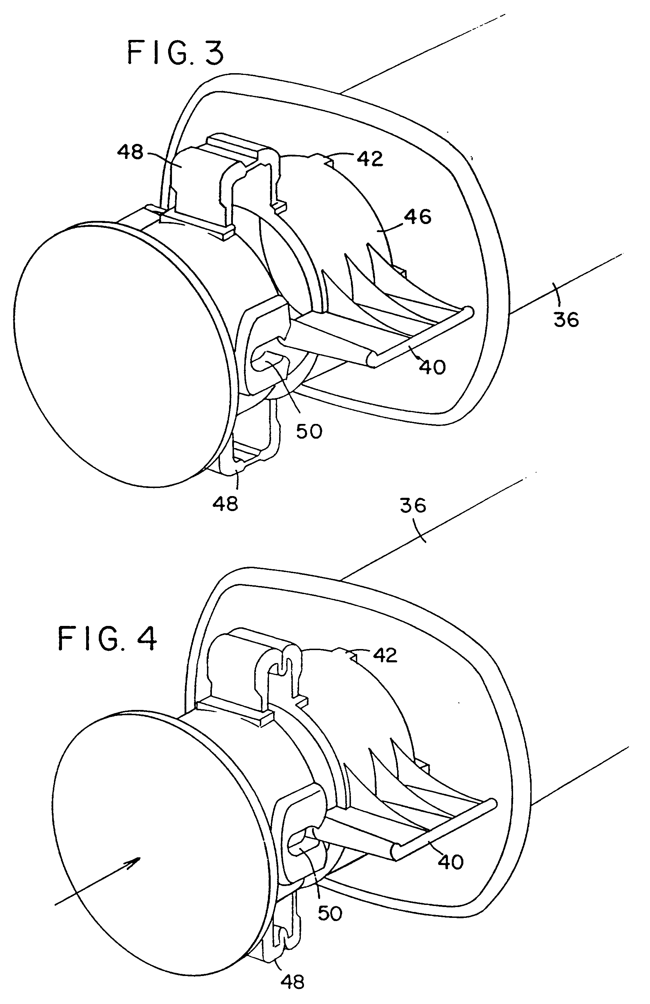

Figure 3 is a perspective view of the cap illustrated in Figure 1.

Figure 4 is a perspective view illustrating the direction that force is applied to the cap to activate the cartridge-needle unit.

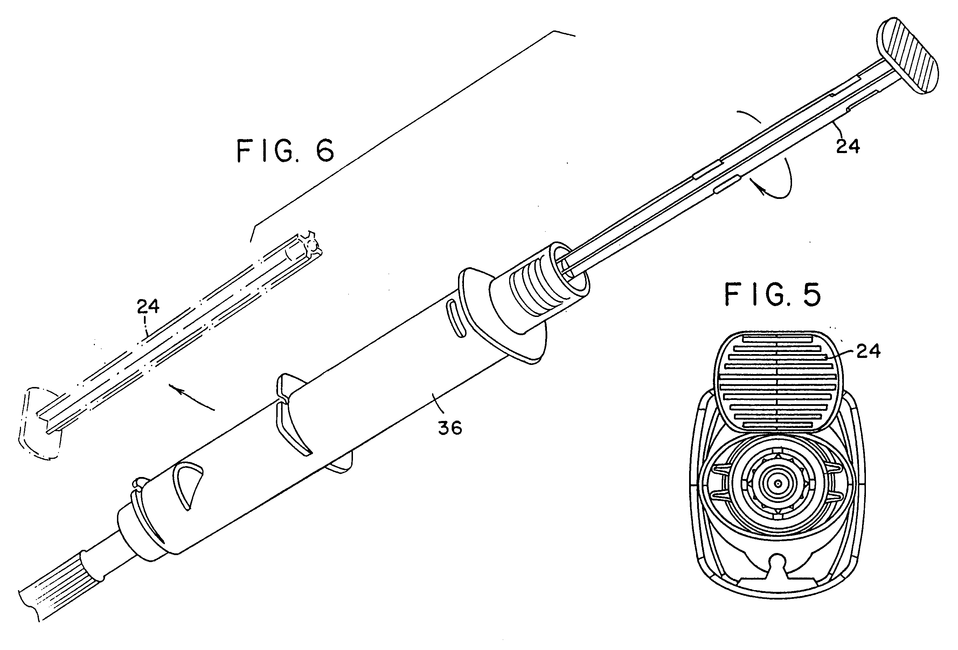

Figure 5 is a top view of the holder depicted in Figure 1.

Figure 6 is a perspective view illustrating a plunger rod unsnapped from the body of the holder and the motion used to attach the plunger rod to the plunger.

Figures 7 and 8 are perspective views of a user removing the cap from the body of the holder.

Figure 9 is a perspective view depicting a needle, an injection site and a user applying force to the plunger rod via the plunger rod thumb pad.

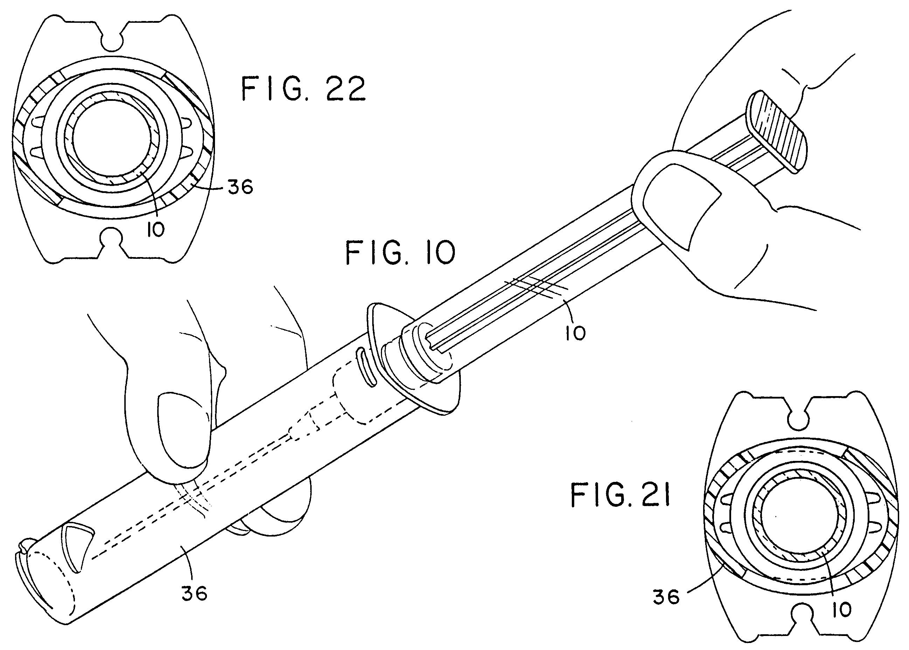

Figure 10 is a perspective view depicting a user grasping the body behind the retraction finger guards and pulling the cartridge-needle unit such that the needle is withdrawn into the body of the holder.

Figures 11 and 12 are perspective and bottom views of a preferred plunger rod for use with the holder of this invention.

Figures 13 and 14 are cross-sectional views depicting the plunger rod of Figure 11 before and after attachment to a plunger.

Figures 15 and 16 are partial cross-sectional front views illustrating a holder of this invention and an associated cartridge-needle unit before and after activation.

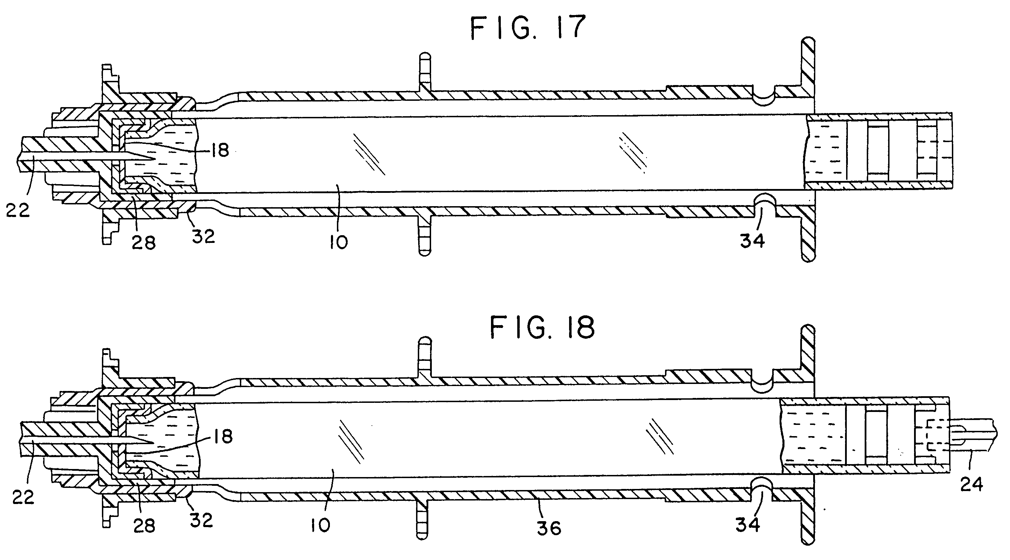

Figures 17 and 18 are partial cross-sectional front views depicting the holder of Figure 1 after removal of the cap, before and after attachment of the plunger rod to the plunger.

Figure 19 is a cross-sectional front view of a holder and cartridge-needle unit having the plunger in a fully expulsed position.

Figure 20 is a partial cross-sectional front view of a needle withdrawn into the body of a holder.

Figures 21 and 22 are cross-sectional top views illustrating the body in undeformed and deformed states respectively.

Figures 23-26 are perspective, top and cross-sectional front and side views of a collar for use with a cartridge-needle unit in a preferred embodiment of the invention.

Figure 27 illustrates a holder featuring a cap comprising a lever element used for cartridge activation and/or cap removal.

Figure 28 depicts a holder comprising male threads and a cap adapted to be threaded thereon for cartridge activation and/or cap removal.

Figure 29 depicts a holder featuring side grooves and triangular cam slots for cartridge activation and/or cap removal.

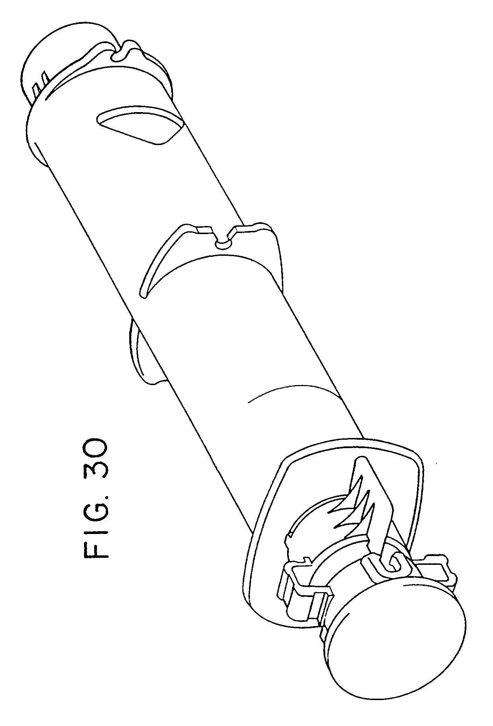

Figure 30 is a perspective view of a holder featuring triangular cam slots.

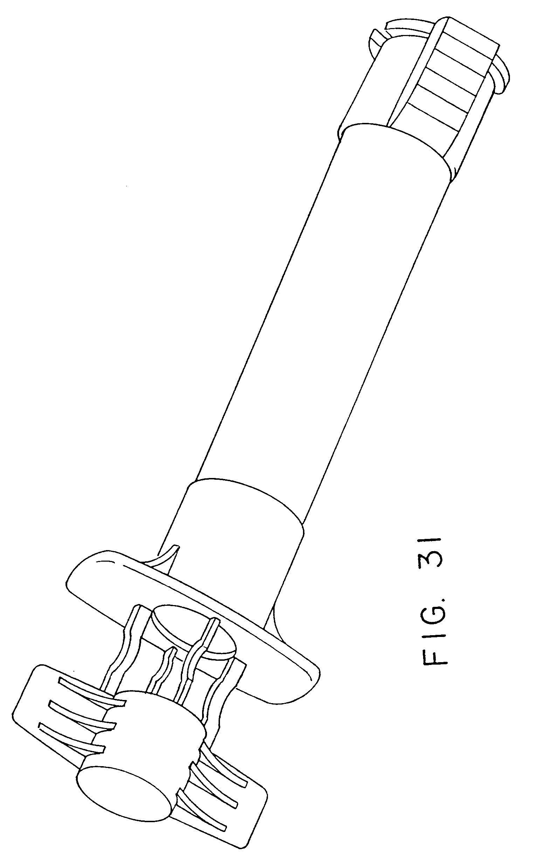

Figure 31 is a perspective view of another holder of the invention featuring a cap attached to the body by tethers which can be severed by application of torque, i.e. a twisting motion to the cap.

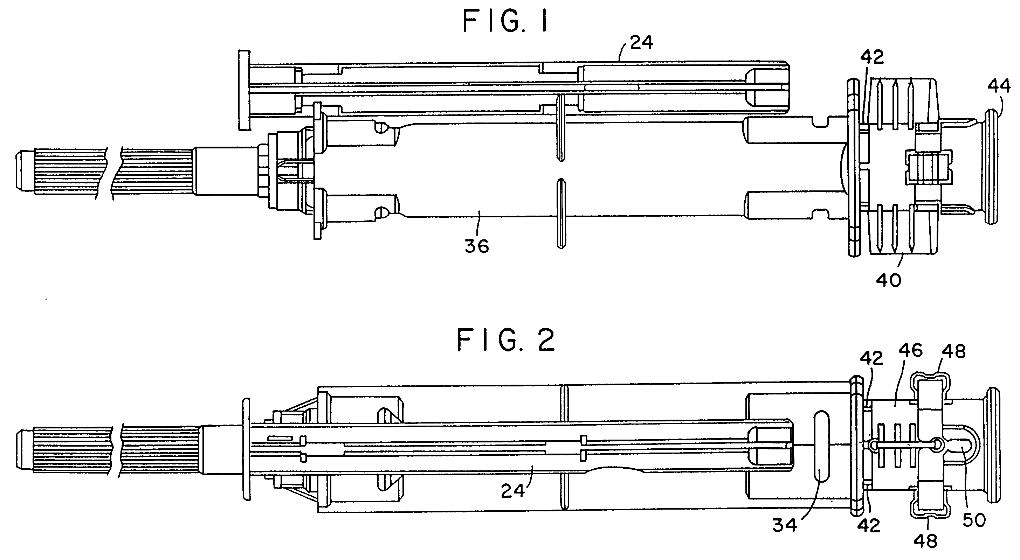

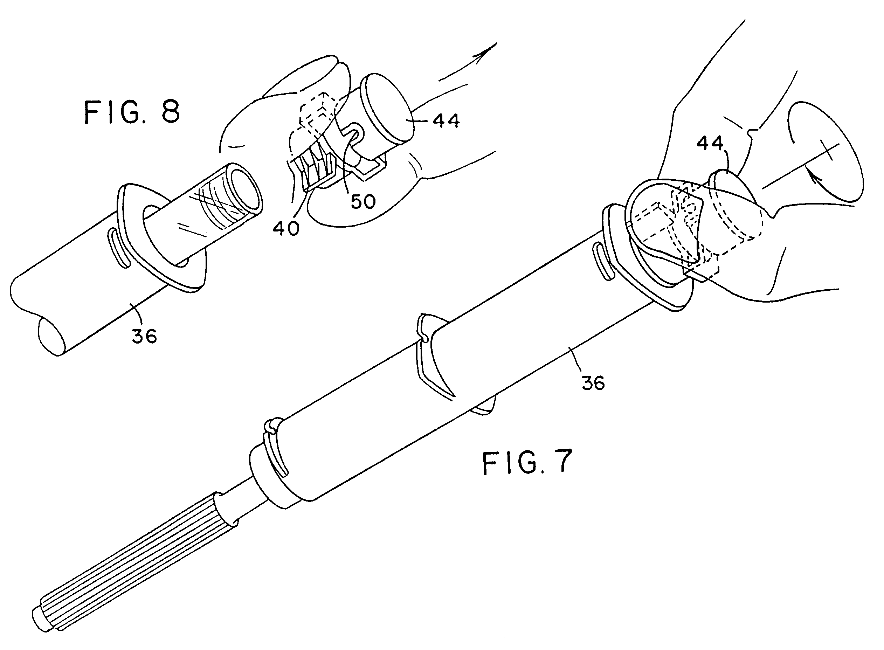

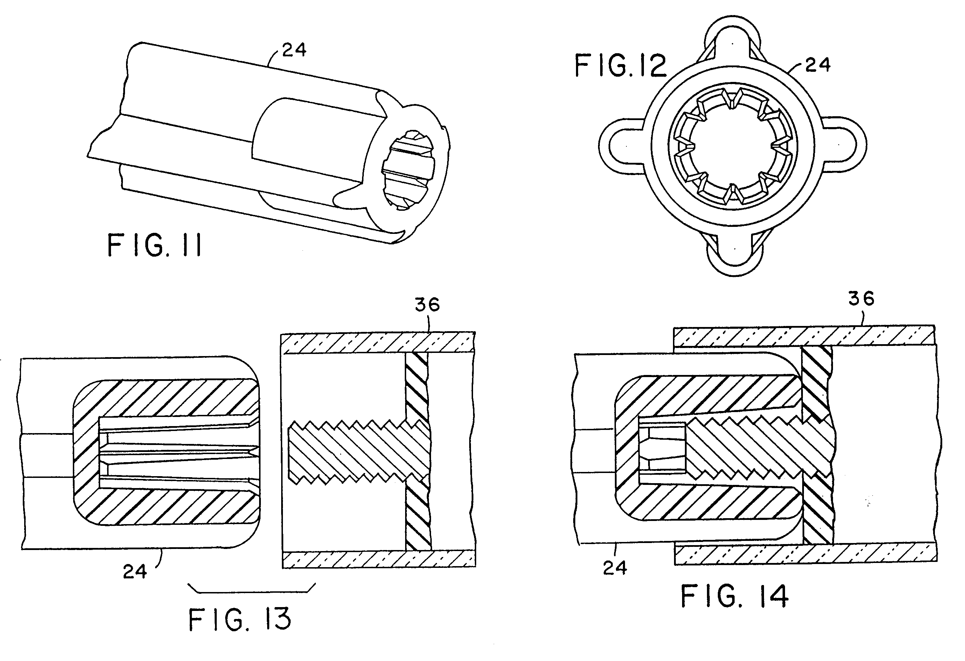

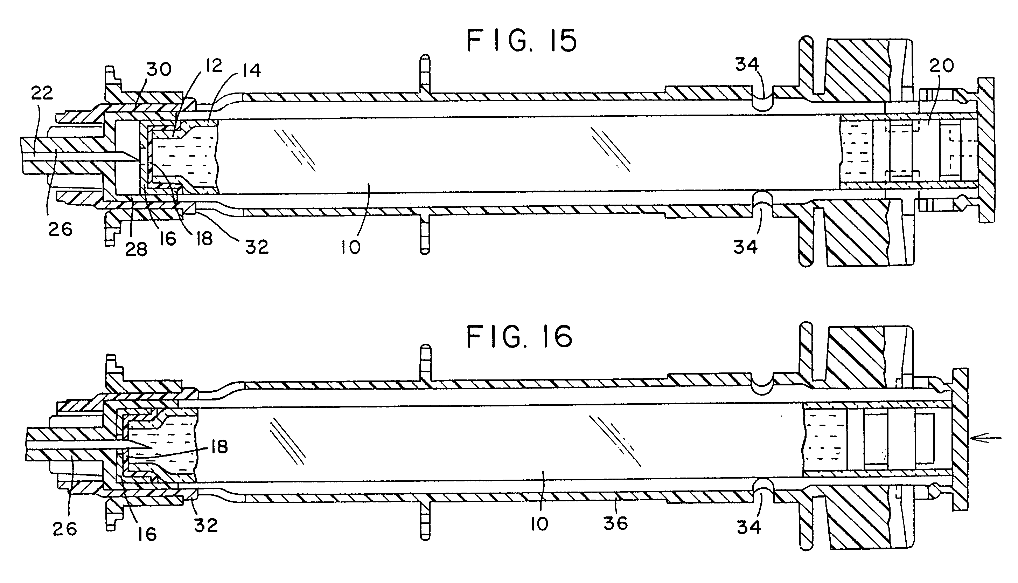

In a preferred embodiment, the holder of this invention is used in conjunction with a prefilled cartridge-needle unit. The prefilled cartridge-needle unit can be of a conventional design and can include a hollow, transparent body, typically fabricated of glass, which is prefilled with a supply of fluid medication or the like. Such cartridge-needle units currently are in widespread commercial use. With reference to Figure 15, the cartridge 10 includes head portion 12 and a cylindrical body 14 which are co-extensively joined together at a relatively narrow neck. A metallic end cap 16 covers a sealed septum 18 which extends across the distal end of the cartridge 10 to prevent contamination and leakage of the fluid contents. Plunger 20 is sized to be received in and slidable axially and reciprocally through the interior of the cartridge 10. The plunger is formed from a relatively dense resilient material, e.g. rubber, and can be moved distally through the cartridge 10 for expulsing the fluid contents of the cartridge via needle cannula 22 so as to project outwardly from the end thereof. The screw-threaded post can be mated to screw-threadable plunger rod 24, Figures 1 and 6, of an associated holder to complete a plunger assembly for controlling the movement of the plunger 20 through the interior of the cartridge 10. The plunger rod 24 can comprise a plurality of ribs extending longitudinally on the interior surface of the cavity adapted to be threaded onto the post. Such a preferred plunger rod is depicted in detail in Figures 11-14. An advantage of this design is that the plunger can be manufactured by conventional injection molding techniques. It is contemplated that other means known in the art can be employed for attaching the rod to the plunger stem.

The necked-down distal end of the cartridge-needle unit can be fitted with a needle hub/needle/needle guard assembly. Needle hub 26 (Figure 15) can be attached to the cartridge 10 by snapping sleeve 28 over the distal end of the cartridge to engage the metallic end cap 16.

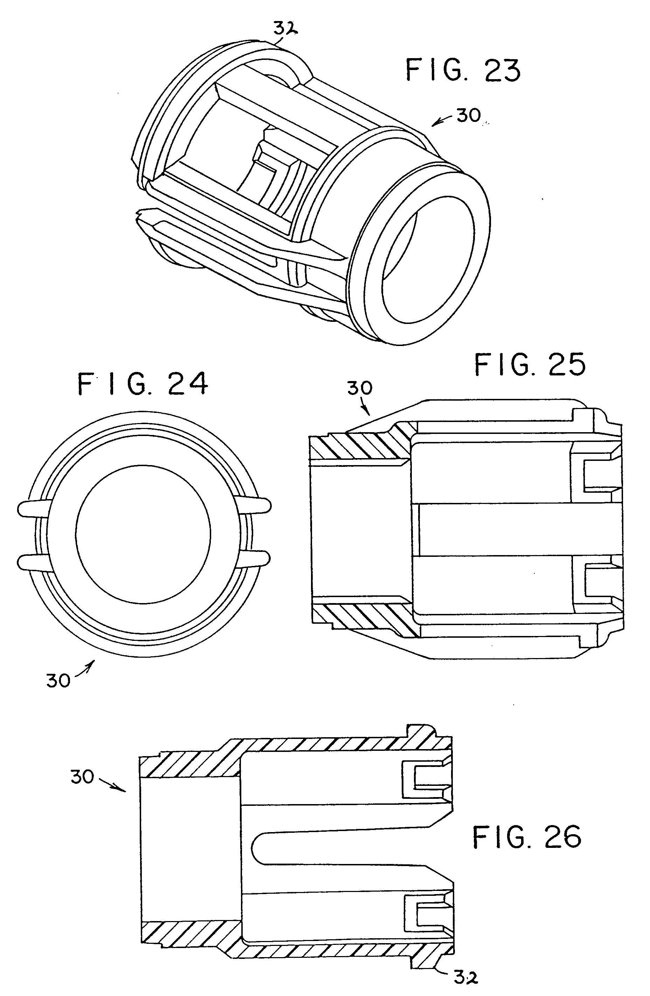

The cartridge-needle unit can be provided with collar 30 extending from the needle end. The collar 30 has a circumferentially extending adapter ring 32 sized to engage specifically positioned pairs of retaining slots 34 formed in the body. In a preferred embodiment, the collar is of the type depicted in Figures 23-26. Alternatively, the cartridge-needle unit can be provided with an integral adapter ring, e.g. as part of the needle hub.

The holder of this invention comprises hollow body 36 (Figure 1) sized for housing the cartridge-needle unit therein. The body can be elliptically deformable, such as is described in PCT/US91/06878. In a preferred embodiment, the holder comprises a hollow body having an elliptical cross-section at the distal end and a circular cross-section at the proximal end.

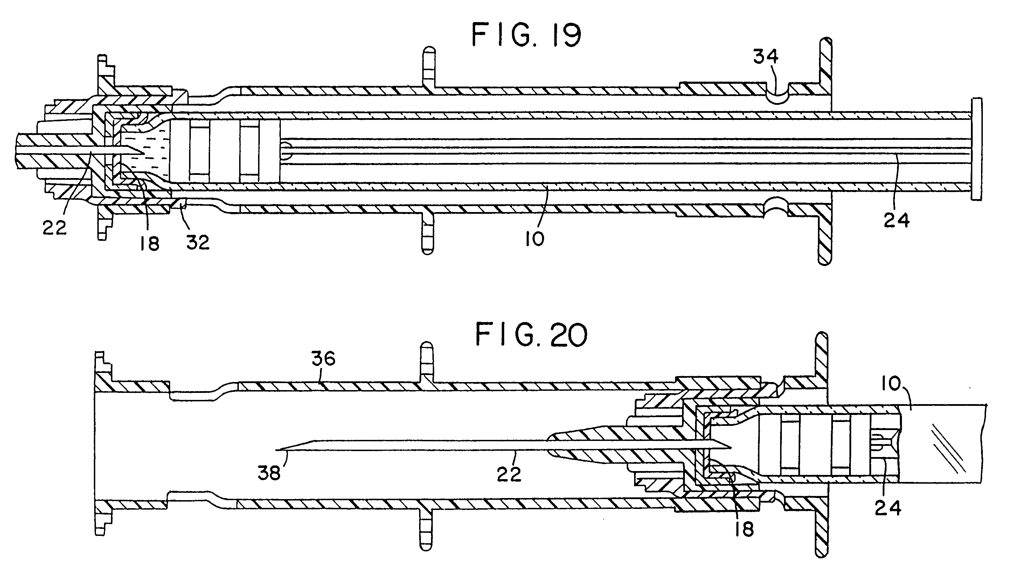

The holder preferably includes means for permitting the body 36 to move axially relative to the cartridge-needle unit. For example, the body 36 can have an elliptical cross-sectional shape with retaining slots 34 positioned through the walls of the body. This permits the outer circumference of the adapter ring 32 to enter retaining slots 34 so long as the body section is in its normal undeformed condition. To disengage the adapter ring 32 from the retaining slots 34, the user squeezes the body 36 inwardly along the major axis of the ellipse thereby permitting its axial movement. Squeeze pads can be provided on the distal end of the body to facilitate the user squeezing in the appropriate orientation relative to the body 36 and retaining slots. The retaining slots 34 can be positioned so that tip 38 (Figure 20) of the needle cannula 22 can be withdrawn within the interior of the body 36 after use.

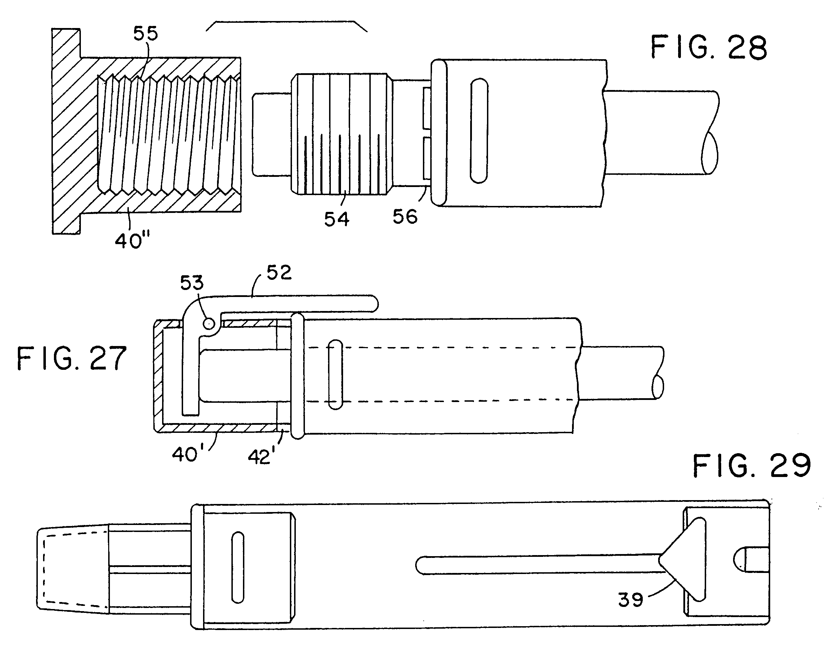

In a preferred embodiment, the retaining slots are cam slots 39. It is a particular advantage that cam slots are self-activating and do not require user interface, i.e. they do not require the user to squeeze the body to release the adapter ring. In a particularly preferred embodiment, cam slots 39 can be triangular in shape as illustrated in Figure 29.

Alternatively, the means for permitting the body to move axially relative to the cartridge-needle unit can comprise side grooves in the body in combination with retaining slots or another positioning member which positions and locks the body. Such grooves facilitate the axial sliding movement of the body relative to the cartridge-needled unit.

In accordance with this invention, the proximal end of the cartridge-needle unit is provided with a cap. The cartridge-needle unit can be activated by application of force to the cap. The force, e.g. a torque can be applied to the cap directly, e.g. by twisting the cap, and/or indirectly, e.g. through a lever element. The cap can be embodied in several different forms.

In a preferred embodiment, cap 40 (Figure 1) is formed integrally with the body 36 and attached to the proximal end thereof by one or more, preferably a plurality of, frangible tethers 42. The cap 40 comprises activation pad portion 44 attached to the portion of the cap containing twist tabs 46 by non-frangible plastic connectors 48 (Figure 2). By selecting an appropriate distance between the pad and tab portions, the cartridge-needle unit can be activated by application of force to the cap, i.e. by pressing the pad portion until it becomes fully seated. The pad portion can include notches 50 which engage the twist tabs to facilitate activation. The twist tabs 46 facilitate application of a sharp twisting motion to the cap by the user in order to fracture the tethers 42, enabling removal of the cap 40. An advantage of this embodiment is that the cap is tamper-evident. When a plurality of tethers are present, one tether can be designed to be stronger than the others, so that the strong tether remains intact when the others are fractured. Accordingly, the cap need not be disposed of as a separate piece.

In another embodiment depicted in Figure 27, cap 40' comprises a plurality of frangible tethers 42' and lever element 52, which can be lifted by the user and rotated about a pivot point 53 to cam the cartridge-needle unit forward and activate the cartridge. After activation, the lever 52 provides force sufficient to fracture the frangible tethers 42', thus separating the cap 40' from the body 36.

In another embodiment illustrated in Figure 28, cap 40'' is provided with female threads 55, and the body 36 is provided with male threads 54 attached through frangible tethers 56. The female threaded cap 40'' is adapted to be threaded onto the male threads 54 of the body 36. To activate the cartridge, the user threads the cap 40'' onto the male threaded body 36 with a twisting motion, driving the cartridge-needle unit forward and activating it. After the cartridge-needle unit is activated, the cap bottoms out, i.e. reaches the end of the thread, and the applied torque fractures the tethers on the body section. The cap can then be discarded and the holder is ready for use.

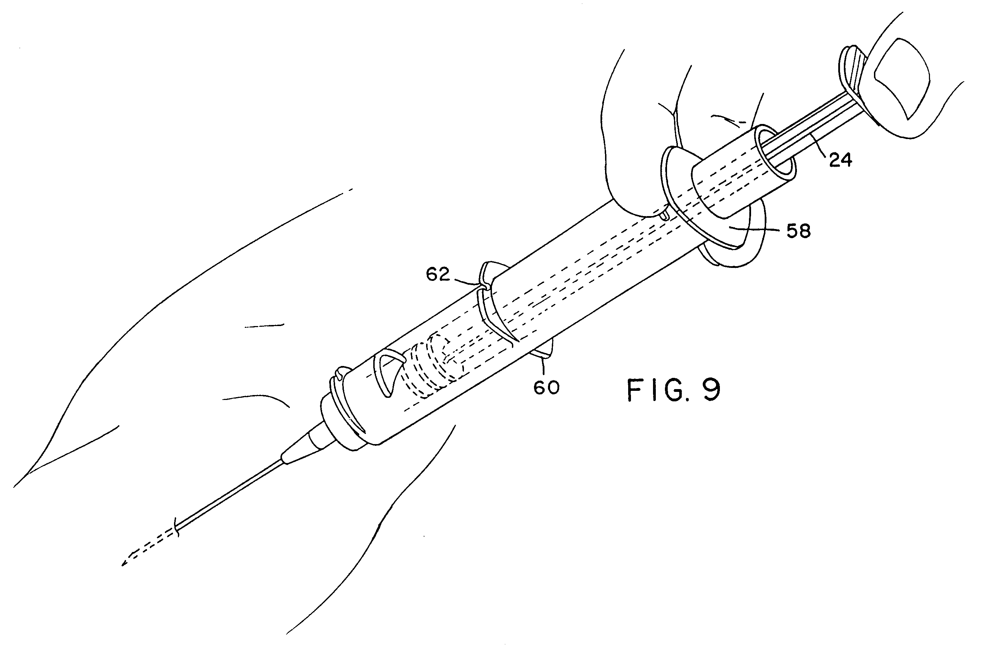

In a preferred embodiment, the holder is provided with finger flange 58 (Figure 9) to facilitate the injection process. When the holder comprises cam slots, it is particularly preferred that retracting finger guards 60 be provided to facilitate retraction of the cartridge-needle unit into the holder in a safe and reliable manner. This combination of features allows the user to keep their fingers a safe distance from the used needle. The body can be provided with retention feature 62 which permits the plunger rod 24 to be attached via a snap fit to the holder.

The syringe holder of this invention can be fabricated of any suitable material including metals and plastics. However, it is well adapted to be made of plastic. In particular, the holder and associated collar can be fabricated of rigid plastic using known precision injection molding techniques. Suitable plastics include polypropylene, polystyrene, polycarbonates, ABS (clear or opaque), nylon, acetals, polyethylene or polyester. The material can include a slip agent or lubricant to facilitate the sliding motion of the body with respect to the cartridge-needle unit. A preferred slip agent is PETRAC SLIPEZE OLEAMIDE, a fatty amide commercially available from Synpro, Cleveland, Ohio.

In use, the holder of this invention operates in conjunction with conventional cartridge-needle units for administration of an injection as follows. First, the plunger rod can be unsnapped from the body of the syringe assembly. The cartridge-needle unit is activated by applying force to the cap. For example, the activation pad can be pressed until it stops and the pad is fully seated. The cap is then removed, for example, by grasping the twist tabs and applying a sharp twisting motion, to expose the plunger. The associated plunger rod can then be attached to the exposed plunger by threading it onto the post and turning clockwise. Next, the needle guard is removed to expose the needle cannula. The needle is inserted into an injection site and an axially and distally directed force is applied by the health care worker to the plunger rod via the actuation button. The distal force is transferred from plunger rod to plunger, to drive the plunger through medicament-containing cartridge-needle unit and to thereby expulse the fluid contents of the cartridge via the needle cannula into the injection site. After the injection is administered, the needle cannula is removed from the injection site. Subsequently, the body can be squeezable deformed, or, when cam slots are present, grasped behind the retraction finger guards, and the cartridge-needle unit can be pulled proximally back by the user such that the exposed needle is withdrawn into the body of the holder, thus significantly reducing the possibility of accidental needle strikes. The holder-used cartridge-needle unit combination can be disposed of in an appropriate manner.

## Claims

1. A disposable holder for use in combination with pre-filled cartridge-needle units having a proximal end and a needle (22) at the distal end thereof, said holder comprising:
a hollow body (36) sized for housing the cartridge-needle unit therein;
means (32,34) for permitting the body (36) to move axially relative to the cartridge-needle unit and for retaining the cartridge-needle unit within the holder; and
a cap (40) for the proximal end of said cartridge-needle unit, characterized in that said cartridge-needle unit can be activated by application of force to said cap (40).

2. A holder as claimed in claim 1 wherein said cap (40) is attached to the proximal end of said body by frangible tethers (42).

3. A holder as claimed in claim 1 wherein said cap (40) comprises a lever element (52).

4. A holder as claimed in claim 1 wherein said body (36) comprises male threads (54), and said cap (40) comprises female threads (55) adapted to be threaded onto said male threads (54).

5. A holder as claimed in any one of the preceding claims, wherein said cartridge-needle unit comprises a circumferential ring (32) and said body (36) is squeezably deformable and comprises retaining slots (34) which accept said circumferential ring (32), positioned so that said needle (22) can be withdrawn into said body (36).

6. A holder as claimed in any one of claims 1 to 4, wherein said cartridge-needle unit comprises a circumferential ring (32) and said body (36) comprises cam slots (39) which accept said ring (32), said slots (39) being positioned so that said needle (22) can be withdrawn into said body (36).

7. A holder as claimed in claim 6 wherein said cam slots (39) are triangular in shape.

8. A disposable holder for use in combination with pre-filled cartridge-needle units having a proximal end and a needle (22) at the distal end thereof, said holder comprising:
a hollow body (36) sized for housing the cartridge-needle unit therein; and
a tamper-evident cap (40) for the proximal end of said cartridge-needle unit attached to the proximal end of said body (36), characterized in that said cartridge-needle unit can be activated by application of force to said cap (40).

9. A holder as claimed in claim 8 wherein said cap (40) is attached to said body by frangible tethers (42).

10. A holder as claimed in claim 8 wherein said cap (40) comprises a lever element (52).

11. A holder as claimed in claim 8 wherein said body (36) comprises male threads (54) and said cap(40) comprises female threads (55) adapted to be threaded onto said male threads (54).
